# EUROPEAN PATENT APPLICATION

(11) **EP 4 019 464 A1**
(43) Date of publication of application: **29.06.2022**
(21) Application number: 20216623.7
(22) Date of filing: 22.12.2020
(51) Int. Cl.: C01B 3/38, C25B 1/04, C07C 29/151

(54) **METHOD AND PLANT FOR PRODUCING SYNGAS**

(71) Applicant: Haldor Topsøe A/S, 2800 Kgs. Lyngby (DK)
(72) Inventor: MORTENSEN, Peter Mølgaard, DK-4000 Roskilde (DK); BØGILD HANSEN, John, 3050 Humlebæk (DK)
(74) Representative: Topsoe A/S

(57) **Abstract**

The present invention describes a method of combining electrolysis, preferably SOEC with reforming, preferably eSMR, to produce a carbon monoxide (CO) rich synthesis gas, providing several synergies and overcoming some limitations of the SOEC technology.

## Description

### Field of Application

The present invention relates to an improved process and plant for generating synthesis gas.

### Background Art

There is presently a rapidly growing interest in using renewable power to produce fuels and chemicals. For carbon containing products like for instance methanol or hydrocarbons the feedstocks can then be CO₂, water and power.

The synthesis gas can then most simply consist of CO₂ and hydrogen generated by electrolysis of water.

If the synthesis gas also comprises carbon monoxide it will be more reactive for both kinetic and thermodynamic equilibrium reasons. In the case of Fischer-Tropsch synthesis, using cobalt based catalysts, CO₂ is indeed not reactive and cannot be used as feedstock.

A CO containing gas can be prepared by reacting hydrogen with CO₂ in a reverse water gas shift (RWGS) reactor according to:

CO₂+ H₂ = CO+ H₂O (1)

Whereby the "surplus" oxygen is removed in the form of water. The RWGS reaction is mildly endothermic and should therefore be carried out at elevated temperature, in order to achieve a reasonable conversion. The eSMR technology platform developed by Haldor Topsoe A/S (HTAS) provides an excellent way of carrying out the RWGS.

A carbon monoxide containing synthesis gas can, however, also very efficiently be produced by co-electrolysis of CO₂ and steam in a Solid Oxide Electrolyser Cell stack.

The present invention therefore describes a method of combining electrolysis, preferably SOEC and reforming, preferably eSMR technology, to produce a carbon monoxide (CO) rich synthesis gas, preferably comprising more than 5 mole% of CO, providing several synergies and overcoming some limitations of the SOEC technology, as described in the next sections. By carbon monoxide rich synthesis gas, carbon monoxide rich syngas or carbon monoxide rich second process stream (5) is understood a gas mixture preferably comprising at least CO and H₂ in a H₂/CO ratio of 4 or below, such as 3, 2, or 1.

Document EP 2 491 998 discloses a method for the production of synthesis gas from CO₂ and water with the help of electrical energy, hydrogen being first generated by steam electrolysis, which is then partly used to convert CO₂ according to the reverse water gas shift reaction (RWGS reaction) and generate CO.
Document EP 2 491 998 B1 does not disclose operational limits for the high temperature co-electrolysis as it is only dealing with steam electrolysis neither does it address the risk of carbon formation in the electrically heated reverse water gas shift (RWGS) reactor or indeed the mentioned feed/effluent recuperator after the RWGS reactor as the present invention, which provides for elimination of the carbon problems as well as minimizes the content of methane formed in the process.

Document EP3472370 discloses a synthesis gas generation arrangement for generating synthesis gas from CO₂ and H₂O with co-electrolysis and with the corresponding synthesis gas generation method with at least one electrolysis stack. A high-temperature electrolysis cell, namely a Solid Oxide Electrolysis Cell (SOEC), for the generation of H₂- and CO-containing gases from H₂O and CO₂ through electrolysis (co-electrolysis) works typically at maximum process temperatures of approx. 850-865 °C. Higher process temperatures are not possible with an SOEC, mainly for reasons of material technology. It is mentioned in this document that in addition to the degree of H₂O and CO₂ decomposition of the electrolysis, the quality of the gas produced is primarily influenced by the chemical equilibrium determined by temperature and pressure. Any further influencing of the gas quality in co-electrolysis is not discussed.
Doc EP3472370B1 does not disclose a synergy in operating an eSMR directly sequential to an SOEC as the present invention, which provides for solving carbon formation problems otherwise limiting the operating regime as well as converting the methane which may form in the SOEC .

In particular, the use of the eSMR enables establishment of the reverse water gas shift and methane steam reforming at high temperatures and the use of the downstream boiler eliminates any metal dusting problems cooling the CO rich gas.

### Brief Description of Drawings

Figure 1 shows a general overview of the plant and method of the present invention. Reference numbers are the following:
   (1) First feed stream
   (2) First process stream
   (3) CO₂ stream upstream to the reformer
   (4) Second feed stream
   (5) Second process stream
   (6) CO₂ stream upstream to the electrolyzer
      (A) Electrolyzer, e.g. SOEC
      (B) Reformer, e.g. eSMR
      (C) Heat exchanger, e.g. Boiler
Figure 2 shows a preferred embodiment of the present invention, where a carbon comprising stream (or C stream or carbon import stream) is added which can be used as co-feed to the carbon rich stream (3).
Figure 3 shows another preferred embodiment of the present invention, where a carbon comprising stream (or C stream or carbon import stream) is added , optionally pretreated and then splitted between streams (3) and (6).
Figure 4 shows an example for the general overview provided in Figure 1, where the synthesis gas is used for production of methanol.

### Definitions

**Boudouard reaction** is the redox reaction of a mixture of carbon monoxide and carbon dioxide at a given temperature. It is the disproportionation of carbon monoxide into carbon dioxide and graphite or its reverse: 2CO ⇄ CO₂ + C

The Boudouard reaction to form carbon dioxide and carbon is exothermic at all temperatures. However, the standard enthalpy of the Boudouard reaction becomes less negative with increasing temperature. While the formation enthalpy of CO₂ is higher than that of CO, the formation entropy is much lower. Consequently, the standard free energy of formation of CO₂ from its component elements is almost constant and independent of the temperature, while the free energy of formation of CO decreases with temperature. At high temperatures, the forward reaction becomes endergonic, favoring the (exergonic) reverse reaction toward CO, even though the forward reaction is still exothermic.

The implication of the change in the equilibrium constant with temperature is that a gas containing CO may form elemental carbon if the mixture cools below a certain temperature. The thermodynamic activity of carbon may be calculated for a CO/CO₂ mixture by knowing the partial pressure of each species and the value of the equilibrium constant. For instance, in a high temperature reducing environment, carbon monoxide is the stable oxide of carbon. When a gas rich in CO is cooled to the point where the activity of carbon exceeds 1.0, the Boudouard reaction can take place. Carbon monoxide then tends to disproportionate into carbon dioxide and graphite. In industrial catalysis, carbon formation (also called coking) can cause serious and even irreversible damage to catalysts and catalyst beds or heat exchange equipment.

**Co-electrolysis** means simultaneous electrolysis of CO₂ and H₂O. An SOEC can electrolyze carbon dioxide (CO₂) to carbon monoxide (CO). If water is electrolyzed at the same time, a mixture of hydrogen and CO is produced. This mixture, called syngas, is the starting point of a large number of syntheses of hydrocarbons in the chemical industry. In this way, liquid transport fuels can be produced synthetically. If the electricity is generated by wind turbines or solar cells, the use of the fuel is CO₂ neutral.

**Carbon monoxide (CO) rich** synthesis gas preferably comprises more than 5 mole% of CO and comprises at least H₂ and CO in a preferred H₂/CO ratio of 4 or below, most preferably 3, 2, or 1.

**Carbon dioxide (CO₂) rich** stream, such as stream (3) or (6), preferably comprises at least 25 mole% or at least 30 mole% or at least 35 mole% or at least 40 mole% or at least 45 mole% or at least 50 mole% or at least 55 mole% or at least 60 mole% or at least 65 mole% or at least 70 mole% or at least 75 mole% or at least 80 mole% or at least 85 mole% or at least 90 mole% or at least 95 mole% or up to 100 mole% CO₂. Stream (3) preferably comprises a higher relative percentage of CO₂ than stream (6). Said CO₂ rich stream (3,6) can merge with a carbon import stream or C stream and thereby incorporate other components, different from CO₂.

**Carbon import stream** or **C stream** results from byproducts of a synthesis process arranged in combination with the present invention, e.g., Fischer-Tropsch synthesis or methanol synthesis. Depending on its origin, the C stream may comprise hydrocarbons, CO₂, CO, H₂, CH₄, alcohols, ketones and/or other byproducts of said synthesis process.

**"Heat exchanger"** means a system used to transfer heat between two or more fluids. Heat exchangers are used in both cooling and heating processes. The fluids may be separated by a solid wall to prevent mixing or they may be in direct contact. In particular, "heat exchanger" means a Boiler, by which is understood a mechanical construction where hot gas can heat exchange with liquid water, and this way the hot gas can be cooled while the liquid water is evaporated as steam. Such a configuration is advantageous for fast cooling of a gas because of the high heat transfer numbers which can be achieved.

**Hydrogen rich stream** such as the first process stream (2) preferably comprises at least 25 mole% or at least 30 mole% or at least 35 mole% or at least 40 mole% or at least 45 mole% or at least 50 mole% or at least 55 mole% or at least 60 mole% or at least 65 mole% or at least 70 mole% or at least 75 mole% or at least 80 mole% or at least 85 mole% or at least 90 mole% or at least 95 mole% or up to 100 mole% of H₂.

**Electrolysis** means a technique that uses direct electric current (DC) to drive an otherwise non-spontaneous chemical reaction. Electrolysis is commercially important as a stage in the separation of elements from naturally occurring sources such as ores using an electrolytic cell. The voltage that is needed for electrolysis to occur is called the decomposition potential.

**eSMR** means an electrically heated reformer. The electrically heated reformer preferably comprises a pressure shell housing a structured catalyst, wherein the structured catalyst comprises a macroscopic structure of an electrically conductive material. The macroscopic structure supports a ceramic coating, where said ceramic coating supports a catalytically active material. The reforming step in this aspect comprises the additional step of supplying electrical power via electrical conductors connecting an electrical power supply placed outside said pressure shell to said structured catalyst, allowing an electrical current to run through said macroscopic structure material, thereby heating at least part of the structured catalyst to a temperature of at least 500°C.

Suitably, the electrical power supplied to the electrically heated reformer is generated by means of a renewable energy source.

The structured catalyst of the electrically heated reformer is configured for steam reforming. This reaction takes place according to the following reactions:

CH₄ + H₂O ↔ CO + 3H₂

CH₄ + 2H₂O ↔ CO₂ + 4H₂

CH₄ + CO₂ ↔ 2CO + 2H₂

The structured catalyst is composed a metallic structure, a ceramic phase, and an active phase. The metallic structure may be FeCr Alloy, Alnico, or similar alloys. The ceramic phase may be Al₂O₃, MgAl₂O₄, CaAl₂O₄, ZrO₂, Yttrium oxides, or a combination thereof. The catalytically active material may be Ni, Ru, Rh, Ir, or a combination thereof.

In an embodiment, the macroscopic structure(s) has/have a plurality of parallel channels, a plurality of non-parallel channels and/or a plurality of labyrinthic channels. The channels have walls defining the channels. Several different forms and shapes of the macroscopic structure can be used as long as the surface area of the structured catalyst exposed to the gas is as large as possible.

**Feed/effluent exchanger** means a heat exchanger exchanging heat between the inlet and outlet of a device and is herein used to preheat the feed gas to the SOEC/electrolyzer.

**Metal dusting/carbon formation** means a form of corrosion that occurs when susceptible materials are exposed to environments with high carbon activities. The corrosion manifests itself as a break-up of bulk metal to metal powder. The suspected mechanism is firstly the deposition of a graphite layer on the surface of the metal, usually from carbon monoxide (CO) in the vapour phase. This graphite layer is then thought to form metastable M₃C species (where M is the metal), which migrate away from the metal surface. However, in some regimes no M₃C species are observed indicating a direct transfer of metal atoms into the graphite layer. The temperatures normally associated with metal dusting are high (300-850 °C). From a general understanding of chemistry, it can be deduced that at lower temperatures, the rate of reaction to form the metastable M₃C species is too low to be significant, and at much higher temperatures the graphite layer is unstable and so CO deposition does not occur (at least to any appreciable degree). There are several proposed methods for prevention or reduction of metal dusting; the most common seem to be aluminide coatings, alloying with copper and addition of steam.

**Pure CO** means a gas stream with a concentration of CO >90%, preferably >95%, or even more preferably >98% or up to 100%.

**"Pressure",** P, means gauge pressure and is measured in bar(g). Gauge pressure is the pressure relative to atmospheric pressure and it is positive for pressures above atmospheric pressure, and negative for pressures below it. The difference between bar and bar(g) is the difference in the reference considered. Measurement of pressure is always taken against a reference and corresponds to the value obtained in a pressure measuring instrument. If the reference in the pressure measurement is vacuum we obtain absolute pressure and measure it in bar only. If the reference is atmospheric pressure then pressure is cited in bar(g).

**Reducing agent** (also called a reductant or reducer) is an element or compound that loses (or "donates") an electron to an electron recipient (oxidizing agent) in a redox chemical reaction. A reducing agent is thus oxidized when it loses electrons in the redox reaction. Reducing agents "reduce" (or, are "oxidized" by) oxidizing agents. Oxidizers "oxidize" (that is, are reduced by) reducers.

**Reforming technology** is to be understood as a chemical reaction technology suitable for producing synthesis gas comprising CO and H₂, typically from gas mixtures comprising methane and steam. Said reforming technology facilitates the endothermic reaction between CH₄ and H₂O according to: CH₄ + H₂O ⇔ CO + 3H2, and typically also the water gas shift reaction will be facilitated according to CO + H₂O ⇔ CO₂ + H₂. Specific embodiments of such technology include tubular reforming also known as SMR technology, typically utilizing external heating of tubes filled with catalyst to facilitate the reactions. Other embodiments include heat exchange type reformers, where a hot process gas is used to heat typically reactor tubes filled with catalyst particles. Other embodiments include electrically heated reactors, such as eSMR, where electricity is used as energy input for the endothermic reactions. Resistance heating or induction heating have both been demonstrated as technical solutions in this regard.

**SOEC** means a solid oxide electrolyzer cell, i.e., a solid oxide fuel cell that runs in regenerative mode to achieve the electrolysis of water (and/or carbon dioxide) by using a solid oxide, or ceramic, oxygen ion conducting electrolyte to produce hydrogen gas (and/or carbon monoxide) and oxygen.

**SMR** means steam methane reforming and is a method for producing syngas (hydrogen and carbon monoxide) by reaction of hydrocarbons with water. Commonly natural gas is the feedstock. The main purpose of this technology is hydrogen production. The reaction is represented by this equilibrium: CH₄ + H₂O ⇄ CO + 3 H₂
The reaction is strongly endothermic (consumes heat, ΔHr= 206 kJ/mol) and is conducted in a reformer vessel where a high pressure mixture of steam and methane are put into contact with typically a nickel catalyst. Catalysts with high surface-area-to-volume ratio are preferred because of diffusion limitations due to high operating temperature.
Via the water-gas shift reaction, additional hydrogen can be obtained by treating the carbon monoxide generated by steam reforming with water: CO + H₂O ⇄ CO₂ + H₂
This reaction is mildly exothermic (produces heat, ΔHr= -41 kJ/mol).

### Description

The present invention refers to a method for producing syngas rich in CO, preferably by combining at least one SOEC with at least one eSMR and at least one boiler. The present invention also refers to a plant to operate said method.

The method for producing synthesis gas comprising CO, according to the present invention, comprises the following steps:
a) a first feed stream (1) comprising steam and hydrogen is partially converted to a hydrogen rich first process stream (2) by electrolysis (A);
b) first process stream (2) originating a second feed stream (4) which is converted to a CO rich second process stream (5) in a reforming step (B);
c) said second process stream (5) comprising carbon monoxide rich syngas and steam is cooled (C), providing another stream comprising steam which directly or indirectly enters said first feed stream (1), wherein the molar H₂ to CO ratio in said second process stream (5) is below 4.5 and wherein at least one of i) first feed stream (1) or ii) first process stream (2) is mixed with a CO₂ rich stream (3,6).

In a particularly preferred embodiment, a first feed stream (1) comprising steam and hydrogen is mixed with a CO₂ rich stream (6) and then partially converted to a hydrogen rich first process stream (2) by electrolysis (A);
In another particularly preferred embodiment, the first process stream (2) is mixed with a CO₂ rich stream (3) and then originates a second feed stream (4) which is converted to a CO rich second process stream (5) in a reforming step (B);
In another particularly preferred embodiment, both first feed stream (1) and first process stream (2) are mixed with a CO₂ rich stream (3,6).

First feed stream (1) may also comprise CO. In particular regarding step c), by 1) directly is most preferably understood that the steam stream from the heat exchanger enters directly to the first feed stream and by 2) indirectly is most preferably understood that the steam is first collected, in e.g. a steam header, and steam then enters the first feed stream regulated from the steam header.

Regarding the method of the present invention, although co-electrolysis by SOEC is a very efficient synthesis gas generation technology, currently the application of the most cost effective cells using Ni-cermets as cathodes has two problems:
1) The conversion of especially CO₂ per pass must be limited due to the risk of carbon formation in the stacks, due to especially the Boudouard reaction, but also the CO reduction reaction, aggravated by diffusion limitations in the electrodes and simultaneously,
2) production of methane in the cathode compartment, which is an inert gas in downstream syntheses necessitating higher operating pressures and higher loss in purge gas.

Another problem is :
3) a potential for metal dusting/carbon formation in the feed/effluent exchanger typically used to preheat the feed gas to the SOEC.

By adding at least a part of the CO₂ feed after the, at least one, SOEC (A) directly to an, at least one, eSMR (B) arranged downstream, e.g., preferably arranged in series with an SOEC, these problems can be overcome as the final CO₂ conversion occurs at elevated temperature far above the Boudouard (and CO reduction) temperature in the eSMR monoliths where diffusion restrictions also are absent. Additionally, the eSMR will ensure that practically all methane generated in the SOEC will be converted into synthesis gas. The metal dusting/carbon formation risk in heat exchanger (C), preferably a boiler, is avoided by instantaneous cooling of the synthesis gas in a boiler, which in contrast has the added synergy that it can generate the steam for the SOEC feed.

In most of the literature the above problems have not been addressed, but alternative solutions to the above mentioned problems would be:
1) to limit the conversion in the SOEC which will increase the energy consumption because the feed water needs to be reevaporated more.
2) could be solved by using all ceramic cathodes and heat exchangers but these are not cost effective, at least for now.
3) can be alleviated at least to some extent by using expensive alloys in the feed/effluent exchanger.
However, the above alternative solutions will still have an increased byproduct formation of methane.

A preferred embodiment of a plant according to the present invention comprises an SOEC with an electrical preheater for the fuel feed side and a feed effluent exchanger on the air side. The SOEC will operate from approximately 700 to 825°C. All or part of the CO₂ is then added to an eSMR, preferably in series with the SOEC. The eSMR will operate at exit temperatures of 950 to 1050 °C. After the eSMR the exit gas is cooled in a steam generator providing feed stock steam to the SOEC. Subsequently some of the residual enthalpy in the fuel and air streams are recuperated to be used in the downstream synthesis unit and finally the fuel gas is cooled down to close to ambient temperature and the nonconverted water is condensed and reused. Part of the generated syngas is recycled back to the SOEC in order to prevent oxidation of the nickel electrodes.

The present invention also refers to syngas produced by operating the method herein described in a plant according to the present invention, enabling Fischer Tropsch synthesis and/or methanol synthesis, which then can be carried out at a much lower pressure than using a more CO₂ rich gas (i.e. when compared to the case of methanol synthesis directly from a feedstock of H₂ and CO₂). The use of the eSMR enables establishment of the reverse water gas shift and methane steam reforming equilibrium at high temperatures, higher than what is achievable in the SOEC, and the use of the downstream boiler eliminates any metal dusting problems, cooling the CO rich gas.
The process of the invention can be used to generate practically any synthesis gas with any H₂/CO ratio. This ratio could be 4, 3, 2, or 1 and even lower. The process can also be used to make a designed module for methanol, close to the stoichiometric requirement of (H₂-CO₂)/(CO+CO₂) = 2 which is rich in CO.
The syngas can also be used for Fischer-Tropsch synthesis where the H₂/CO ratio should be close to 2, such as 1.95.

Also, synthesis gas for oxo-alcohols are possible, where the H₂/CO ratio should be close to 1.0.

Another use of the process could be for production of reducing synthesis gas, as used e.g. for iron ore reduction. Here the synthesis gas module should be tailored to a ratio of (CO+H₂)/(CO₂+H₂O) > 7.5.
The combination of SOEC for oxygen extraction, combined with eSMR for high temperature syngas is in this context very attractive for reduction purposes.
In general, the process is advantageously combined with synthesis gas separation steps to produce substantially pure streams of CO and H₂ for use in synthesis applications. Substantially pure CO finds many uses within the polymer industry, especially for production of phosgene, an important intermediate in some polymerization reactions, but also for chemicals production such as acetic acid from CO and methanol.

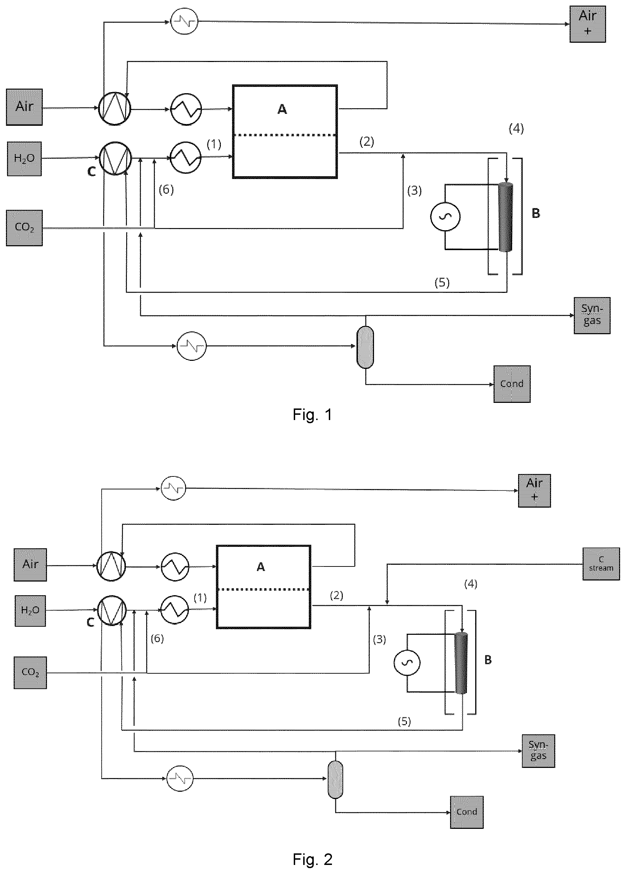

In a preferred embodiment of the present invention (Figure 2), a carbon comprising stream (or C stream) is formed which can be used as co-feed to the carbon rich stream (3) as a carbon import stream. For instance, in the case of Fischer-Tropsch synthesis, a byproduct of hydrocarbons is formed and this carbon import stream can be mixed into CO₂.
Such use of C stream (Figure 2) may also arise from methanol synthesis, providing a byproduct of synthesis gas such as 1) mixtures comprising CO, H₂, N₂ and/or CH₄, and/or byproducts such as 2), alcohols, ketones and similar functional hydrocarbons, wherein different conditions apply for mixing said byproducts or C streams with the CO₂ rich stream(s).

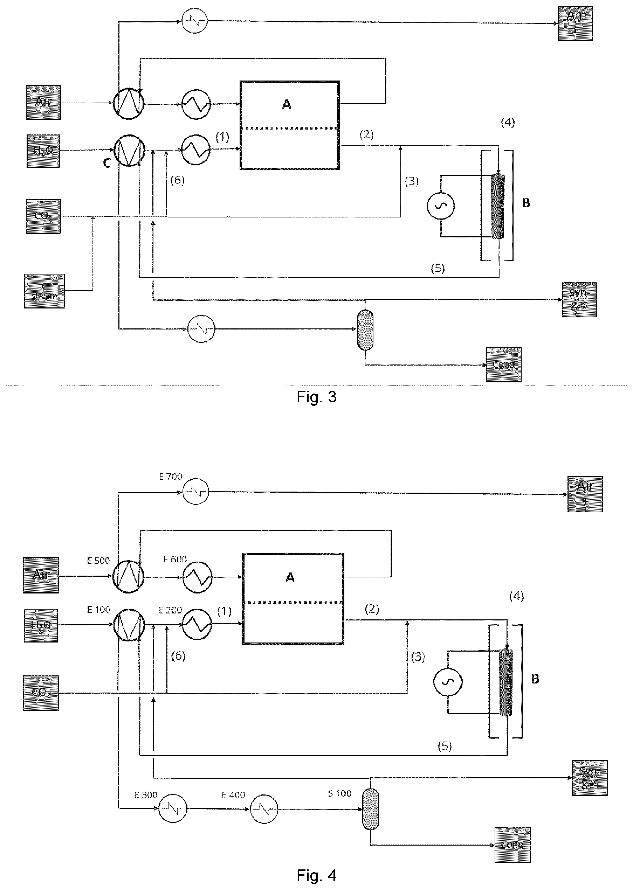

Alternatively, or additionally, in another preferred embodiment of the present invention (Figure 3), such carbon import stream or C stream from Fischer-Tropsch synthesis or from methanol synthesis is optionally pretreated and then divided between streams (3) and (6).

Ways of pretreating said byproducts can include hydrogenation, sulfur removal, methanation, and/or pre-reforming.

### Example 1

The current example is not limiting to the scope of the present invention and provides a particular embodiment of the invention for generation of synthesis gas suitable for production of methanol. The flow scheme is shown on Fig 4.

A boiler feed water flow of 12298 kg/h is sent to the boiler E 100 where it is evaporated an attains a temperature of 134 °C. After the boiler 824 kg/h of CO2 is added as well 927 Nm3/h of recycled synthesis gas. The combined flow is then preheated to 750 °C in the electrical heater, E 200, before entering the cathode chamber of the Solid Oxide Electrolyzer (SOEC), where 80 % of the steam is converted to hydrogen and 68.2 % of the CO2 is converted to carbon monoxide and methane. The methane content of the gas leaving the cathode is 0.27 mole % and the CO is 3.0 mole %. The Boudouard temperature of the gas leaving the cathode is 612 °C. After the SOEC a further 7414 kg/h of CO2 is added before the gas enters the eSMR. In the eSMR the reverse water gas shift and the steam reforming of methane is performed and the gas is heated to 1000 °C. The carbon monoxide content of the gas Is increased to 16.3 mole % and the methane content reduced to 13 ppm. This hot gas is cooled to 150 °C by generating steam in the boiler E 100. The gas is further cooled to 65 °C in the heat exchanger E 300 and used to preheat boiler feed water before it is finally cooled to 20 °C in the water cooler E 400. The unconverted water is then separated from the gas in the separator, S 100. Part of gas leaving the separator is recycled back to the SOEC and the rest sent to the methanol synthesis. On the anode side of the SOEC 14014 kg/h of dry air is compressed and preheated to 730 °C in the feed/effluent exchanger E 500 and finally to 750 °C in the electrical heater E 600 before entering the anode. Due to the steam and CO2 electrolysis the oxygen content is increased to 50 mole % in the gas leaving the cathode. The cathode exit gas is used to preheat the incoming air in the feed/effluent exchanger, E 500, and is further cooled to 65 °C in the heat exchanger E 700.

### Preferred embodiments

1. Method for producing synthesis gas comprising CO, wherein:
   a) a first feed stream (1) comprising steam and hydrogen is partially converted to a hydrogen rich first process stream (2) by electrolysis (A);
   b) first process stream (2) originates a second feed stream (4) which is converted to a CO rich second process stream (5) in a reforming step (B);
   c) said second process stream (5), comprising carbon monoxide rich syngas and steam, is cooled (C) providing another stream comprising steam which directly or indirectly enters said first feed stream (1), wherein the molar H₂ to CO ratio in said second process stream (5) is below 4.5 and wherein at least one of i) first feed stream (1) or ii) first process stream (2) is mixed with a CO₂ rich stream (3,6).
      In a particularly preferred embodiment, a first feed stream (1) comprising steam and hydrogen is mixed with a CO₂ rich stream (6) and then partially converted to a hydrogen rich first process stream (2) by electrolysis (A);
      In another particularly preferred embodiment, the first process stream (2) is mixed with a CO₂ rich stream (3) and then originates a second feed stream (4) which is converted to a CO rich second process stream (5) in a reforming step (B);
      In another particularly preferred embodiment, both first feed stream (1) and first process stream (2) are mixed with a CO₂ rich stream (3,6).
      First feed stream (1) may also comprise CO. In particular regarding step c), by 1) directly is most preferably understood that the steam stream from the heat exchanger enters directly to the first feed stream and by 2) indirectly is most preferably understood that the steam is first collected, in e.g. a steam header, and steam then enters the first feed stream regulated from the steam header.
2. Method according to embodiment 1 wherein the H₂/CO ratio in said syngas comprising carbon monoxide is between approximately 0,5 and 4,5.
3. Method according to any of embodiments 1 or 2, wherein the first feed stream (1) further comprises CO.
4. Method according to any of embodiments 1 to 3, wherein the first feed stream (1) further comprises a carbon dioxide rich stream (6) which comprises at least 25 mole% or at least 30 mole% or at least 35 mole% or at least 40 mole% or at least 45 mole% or at least 50 mole% or at least 55 mole% or at least 60 mole% or at least 65 mole% or at least 70 mole% or at least 75 mole% or at least 80 mole% or at least 85 mole% or at least 90 mole% or at least 95 mole% or at least 98 mole% or up to 100 mole% CO₂.
5. Method according to any of embodiments 1 to 3 wherein the second feed stream (4) further comprises a carbon dioxide rich stream (3) which comprises at least 25 mole% or at least 30 mole% or at least 35 mole% or at least 40 mole% or at least 45 mole% or at least 50 mole% or at least 55 mole% or at least 60 mole% or at least 65 mole% or at least 70 mole% or at least 75 mole% or at least 80 mole% or at least 85 mole% or at least 90 mole% or at least 95 mole% or at least 98 mole% or up to 100 mole% CO₂.
6. Method according to any of embodiments 1 to 5 wherein stream (3) preferably comprises more CO₂ than stream (6).
7. A method according to any of the previous embodiments wherein the second feed stream (4) also comprises CO, H₂, N₂, CH₄, via merging a carbon import stream with the CO₂ rich stream (3).
8. A method according to any of embodiments 1 to 6, wherein the second feed stream (4) also comprises O₂, hydrocarbons, alcohols and/or ketones, via merging a carbon import stream with the CO₂ rich stream (3).
   Figure 2 shows an overview of embodiments 7 and 8.
9. A method according to any of the previous embodiments wherein the first feed stream (1) also comprises CO, H₂, N₂, CH₄, via splitting a carbon import stream between CO₂ rich streams (3) and (6).
10. A method according to any of embodiments 1 to 8 wherein the first feed stream (1) also comprises O₂, hydrocarbons, alcohols and/or ketones, via splitting a carbon import stream between CO₂ rich streams (3) and (6).
   Figure 3 shows an overview of embodiments 9 and 10.
11. Method according to any of the previous embodiments wherein said first process stream (2), when exiting the electrolyzer, has a temperature from approximately 600 to 1000°C, preferably 700 to 850°C, which is lower than the temperature of said second process stream (5), when exiting the reformer, of approximately 850 to 1200°C, preferably 950 to 1050°C.
12. A method according to any of the previous embodiments, wherein the first feed stream (1) and the second feed stream (4) are heated by means of electrically heating, condensing steam, gas heated heat exchangers, or a combination thereof.
13. Method according to any of the previous embodiments wherein part of the residual enthalpy in the synthesis gas and air streams is recuperated to be used in a downstream synthesis and the second process stream (5) comprising syngas and steam is cooled down approximately to room temperature, the nonconverted water being condensed and reused.
14. Method according to any of the previous embodiments wherein part of the generated syngas is recycled back into the first feed stream (1) to an SOEC.
15. Method according to any of the previous embodiments wherein a syngas separation step is performed after step c) to provide pure streams of CO and H₂.
16. Method according to any of the previous embodiments wherein carbon monoxide (CO) rich synthesis gas, preferably comprises more than 5 mole% of CO and is a gas mixture comprising at least H₂ and CO in a preferred H₂/CO ratio of 4 or below, most preferably 3, 2, or 1.
17. Plant for producing synthesis gas comprising CO, wherein at least one electrolyzer (A) is arranged upstream to at least one reformer (B) such that:
   a) a first feed stream (1) comprising steam and hydrogen is partially converted to a hydrogen rich first process stream (2) in the, at least one, electrolyzer (A);
   b) first process stream (2) originates a second feed stream (4) which is converted to a carbon monoxide rich second process stream (5) in at least one, reformer (B);
   c) said second process stream (5), comprising carbon monoxide rich syngas and steam, is cooled in a heat exchanger (C), providing another stream comprising steam which directly or indirectly enters back into the, at least one, electrolyzer (A), wherein at least one of i) first feed stream (1) or ii) first process stream (2) is mixed with a CO₂ rich stream (3,6).
18. Plant according to embodiment 17 wherein electrolyzer A is an SOEC, reformer (B) is an eSMR and heat exchanger (C) is a boiler.
19. Plant according to any of embodiments 17 or 18 wherein a synthesis unit is downstream to the production of synthesis gas.
20. Plant according to embodiment 19 wherein said synthesis unit is a Fischer-Tropsch synthesis reactor system for producing fuels.
21. Plant according to embodiment 19 wherein said synthesis unit is a methanol reactor system for producing methanol.
22. Syngas obtained by the method according to any of embodiments 1 to 16 in a plant according to any of embodiments 17 to 21, wherein said syngas has a module of (H₂-CO₂)/(CO+CO₂) in the range from 1.8 to 2.2, suitable for methanol synthesis in a downstream methanol reactor system for methanol production.
23. Syngas obtained by the method according to any of embodiments 1 to 16 in a plant according to any of embodiments 17 to 21, wherein said syngas has a ratio of H₂/CO in the range from 1.8 to 2.2, suitable for Fischer-Tropsch synthesis in a downstream Fischer-Tropsch synthesis reactor system for crude oil and/or wax production.
24. Syngas obtained by the method according to any of embodiments 1 to 16 in a plant according to any of embodiments 17 to 21, wherein said syngas has a module of (CO+H₂)/(CO₂+H₂O) > 7.5 and is suitable as a reducing agent.
25. Syngas obtained by the method according to any of embodiments 1 to 16 in a plant according to any of embodiments 17 to 21, wherein said syngas has a ratio of H₂/CO < 1.5 and is suitable as a source of CO.
   This is favorable when the syngas is to be used for pure CO production in e.g. a cold box, or in oxoalcohol synthesis, or for acetic acid production.
26. Use of pure CO obtained according to embodiment 15 to manufacture polymers, wherein the CO is used to produce intermediates in the production schemes, such as phosgene.
27. Use of pure CO obtained according to embodiment 15 to manufacture chemicals, such as acetic acid.

## Claims

1. Method for producing synthesis gas comprising CO, comprising the following steps:
a. a first feed stream (1) comprising steam and hydrogen is partially converted to a hydrogen rich first process stream (2) by electrolysis (A);
b. first process stream (2) originates a second feed stream (4) which is converted to a CO rich second process stream (5) in a reforming step (B);
c. said second process stream (5) comprising CO rich syngas and steam is cooled (C), providing another stream comprising steam which enters said first feed stream (1), wherein the molar H₂ to CO ratio in said second process stream (5) is below 4.5 and wherein at least one of i) first feed stream (1) or ii) first process stream (2) is mixed with a CO₂ rich stream (3,6).

2. Method according to claim 1, wherein the first feed stream (1) further comprises CO.

3. Method according to any of claims 1 or 2, wherein the first feed stream (1) further comprises a carbon dioxide rich stream (6) which comprises at least 25 mole% or at least 30 mole% or at least 35 mole% or at least 40 mole% or at least 45 mole% or at least 50 mole% or at least 55 mole% or at least 60 mole% or at least 65 mole% or at least 70 mole% or at least 75 mole% or at least 80 mole% or at least 85 mole% or at least 90 mole% or at least 95 mole% or at least 98 mole% or up to 100 mole% CO₂.

4. Method according to any of claims 1 or 2, wherein the second feed stream (4) further comprises a carbon dioxide rich stream (3) which comprises at least 25 mole% or at least 30 mole% or at least 35 mole% or at least 40 mole% or at least 45 mole% or at least 50 mole% or at least 55 mole% or at least 60 mole% or at least 65 mole% or at least 70 mole% or at least 75 mole% or at least 80 mole% or at least 85 mole% or at least 90 mole% or at least 95 mole% or at least 98 mole% or up to 100 mole% CO₂.

5. Method according to any of claims 1 to 4 wherein stream (3) preferably comprises more CO₂ than stream (6).

6. A method according to any of the previous claims wherein the second feed stream (4) also comprises CO, H₂, N₂, CH₄, via merging a carbon import stream with the CO₂ rich stream (3).

7. A method according to any of claims 1 to 5, wherein the second feed stream (4) also comprises O₂, hydrocarbons, alcohols and/or ketones, via merging a carbon import stream with the CO₂ rich stream (3).

8. A method according to any of the previous claims wherein the first feed stream (1) also comprises CO, H₂, N₂, CH₄, via splitting a carbon import stream between CO₂ rich streams (3) and (6).

9. A method according to any of claims 1 to 7 wherein the first feed stream (1) also comprises O₂, hydrocarbons, alcohols and/or ketones, via splitting a carbon import stream between CO₂ rich streams (3) and (6).

10. Method according to any of the previous claims wherein said first process stream (2), when exiting the electrolyzer, has a temperature from approximately 600 to 1000°C, preferably 700 to 850°C, which is lower than the temperature of said second process stream (5), when exiting the reformer, of approximately 850 to 1200°C, preferably 950 to 1050°C.

11. A method according to any of the previous claims, wherein the first feed stream (1) and the second feed stream (4) are heated by means of electrically heating, condensing steam, gas heated heat exchangers, or a combination thereof.

12. Method according to any of the previous claims wherein part of the residual enthalpy in the synthesis gas and air streams is recuperated to be used in a downstream synthesis and the second process stream (5) comprising syngas and steam is cooled down approximately to room temperature, the nonconverted water being condensed and reused.

13. Method according to any of the previous claims wherein part of the generated syngas is recycled back into the first feed stream (1) to an SOEC.

14. Plant for producing synthesis gas comprising CO, wherein at least one electrolyzer (A) is arranged upstream to at least one reformer (B) such that:
a) a first feed stream (1) comprising steam and hydrogen is partially converted to a hydrogen rich first process stream (2) in the, at least one, electrolyzer (A);
b) first process stream (2) originates a second feed stream (4) which is converted to a carbon monoxide rich second process stream (5) in at least one, reformer (B);
c) said second process stream (5) comprising carbon monoxide rich syngas and steam is cooled in a heat exchanger (C), providing another stream comprising steam which enters back into the, at least one, electrolyzer (A), wherein at least one of i) first feed stream (1) or ii) first process stream (2) is mixed with a CO₂ rich stream (3,6).

15. Plant according to claim 14 wherein electrolyzer A is an SOEC, reformer (B) is an eSMR and heat exchanger (C) is a boiler.

16. Plant according to any of claims 14 or 15 wherein a synthesis unit is downstream to the production of synthesis gas.

17. Plant according to claim 16 wherein said synthesis unit is a Fischer-Tropsch synthesis reactor system for producing fuels.

18. Plant according to claim 16 wherein said synthesis unit is a methanol reactor system for producing methanol.

19. Syngas obtained by the method according to any of claims 1 to 13 in a plant according to any of claims 14 to 18, wherein said syngas has a module of (H₂-CO₂)/(CO+CO₂) in the range from 1.8 to 2.2, suitable for methanol synthesis in a downstream methanol reactor system for methanol production.

20. Syngas obtained by the method according to any of claims 1 to 13 in a plant according to any of claims 14 to 18, wherein said syngas has a ratio of H₂/CO in the range from 1.8 to 2.2, suitable for Fischer-Tropsch synthesis in a downstream Fischer-Tropsch synthesis reactor system for crude oil and/or wax production.

21. Syngas obtained by the method according to any of claims 1 to 13 in a plant according to any of claims 14 to 18, wherein said syngas has a module of (CO+H₂)/(CO₂+H₂O) > 7.5 and is suitable as a reducing agent.

22. Syngas obtained by the method according to any of claims 1 to 13 in a plant according to any of claims 14 to 18, wherein said syngas has a ratio of H₂/CO < 1.5 and is suitable as a source of CO.
